(19) Europäisches Patentamt
European Patent Office
Office européen des brevets

(11) Numéro de publication : **0 001 029**
**B1**

(12) # FASCICULE DE BREVET EUROPÉEN

(45) Date de publication du fascicule du brevet : **01.09.82**

(51) Int. Cl.³ : **C 07 J 41/00**// C07J43/00, C07D317/72, C07C69/14

(21) Numéro de dépôt : **78400083.8**

(22) Date de dépôt : **30.08.78**
**Le dossier contient des informations techniques présentées postérieurement au dépôt de la demande et ne figurant pas dans le présent fascicule.**

(54) **Procédé de préparation d'oximes en 3 de dérivés stéroïdes.**

(30) Priorité : **31.08.77 FR 7726432**

(43) Date de publication de la demande : **07.03.79 (Bulletin 79/05)**

(45) Mention de la délivrance du brevet : **01.09.82 Bulletin 82/35**

(84) Etats contractants désignés : **BE CH DE GB NL SE**

(56) Documents cités :
**FR A 1 490 590**
**STEROIDS, vol. 23(1) 1974, pages 49-64.**

(73) Titulaire : **ROUSSEL-UCLAF**
**102, route de Noisy Boîte postale no.9**
**F-93230 Romainville (FR)**

(72) Inventeur : **Warnant, Julien**
**13, rue Jacques Dulud**
**F-92200 Neuilly Sur Seine (FR)**
Inventeur : **Jolly, Jean**
**22, rue du Clos d'Orléans**
**F-94120-Fontenay-Sous-Bois (FR)**

(74) Mandataire : **Tonnellier, Marie-José et al**
**102, route de Noisy Boîte postale no 9**
**F-93230 Romainville (FR)**

**EP 0 001 029 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de préparation d'oximes en 3 de dérivés stéroïdes

La présente invention concerne un nouveau procédé de préparation d'oximes en 3 de dérivés stéroïdes ainsi que des produits conçus pour sa mise en œuvre.

L'invention a pour objet un procédé de préparation des composés de formule I :

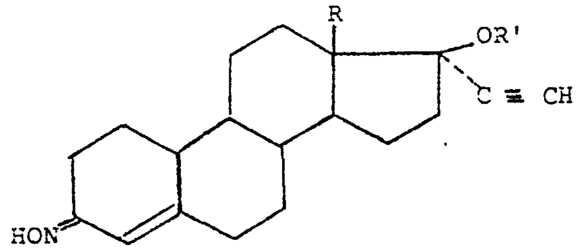

(I)

dans laquelle R représente un radical méthyle ou éthyle et R' un atome d'hydrogène ou un radical acyle renfermant de 2 à 8 atomes de carbone, à partir des composés de formule II :

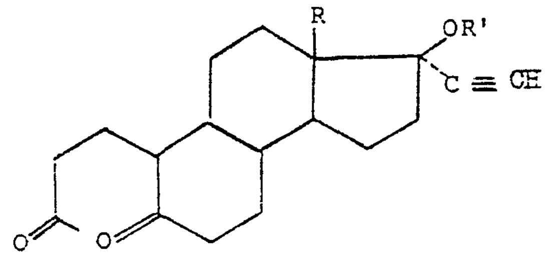

(II)

Par radical acyle, on entend de préférence le reste de l'acide acétique, propionique, butyrique, œnanthylique ou caproïque.

Les composés de formule I sont des produits connus : ils peuvent être préparés par exemple selon le procédé indiqué dans le brevet américain 3 532 689.

La Société demanderesse vient de découvrir un procédé de préparation permettant d'obtenir les produits de formule I par synthèse totale, d'une façon surprenante et avec d'excellents rendements.

L'invention a pour objet un procédé de préparation des composés de formule I :

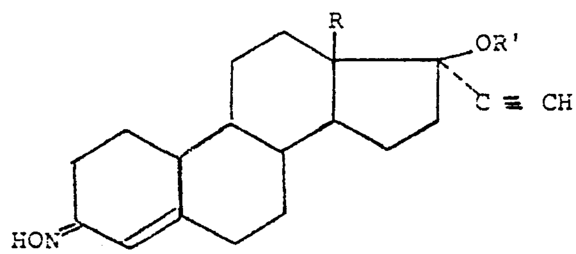

(I)

dans laquelle R et R' sont définis comme précédemment, caractérisé en ce que l'on soumet un composé de formule II :

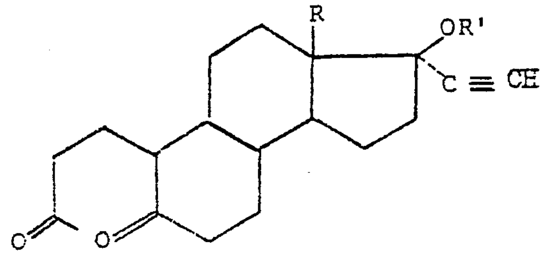

(II)

dans laquelle R et R' conservent leur signification précédente à l'action d'une amine secondaire, puis à l'action d'un acide et enfin à celle de l'hydroxylamine ou d'un sel d'hydroxylamine, pour obtenir le composé recherché correspondant sous forme d'un mélange d'isomères syn et anti, que l'on sépare, si désiré, en chacun des isomères.

L'invention a notamment pour objet un procédé de préparation défini ci-dessus, caractérisé en ce R représente un radical éthyle, et également un procédé caractérisé en ce que R' représente un radical acyle et notamment un radical acétyle.

L'invention a plus particulièrement pour objet un procédé tel que défini ci-dessus, caractérisé en ce que l'on opère en milieu anhydre.

On peut isoler ou ne pas isoler l'énamine obtenue par action de l'amine secondaire sur le composé II et l'invention comprend aussi bien le procédé comportant l'isolement de l'énamine que le procédé ne le comportant pas.

Dans un mode de réalisation préféré de l'invention, on utilise comme amine secondaire la pyrrolidine ou la morpholine et l'on utilise comme acide, l'acide acétique ou l'acide phosphorique.

La séparation des isomères syn et anti est réalisée de préférence selon les techniques usuelles de chromatographie.

Le 3-pyrrolidyl 13 β-éthyl 17 β-acétoxy 17 α-éthynyl gona 3,5-diène isolé éventuellement est un produit nouveau.

Les composés de formule II utilisés comme produits de départ sont des composés connus en général, ils peuvent être préparés par exemple selon le procédé décrit dans le brevet français 1490 590.

Le 3,5-dioxo 4,5-séco 13 β-éthyl, 17 β-acétoxy 17 α-éthynyl gonane utilisé comme produit de départ du procédé de l'invention est un produit nouveau, il peut être préparé par action de l'anhydride acétique sur le 3,5-bis éthylènedioxy 4,5-séco 13 β-éthyl 17 β-hydroxy 17 α-éthynyl gonane, (produit préparé selon le procédé indiqué dans le brevet israélien 28020) et hydrolyse du 3,5-bis éthylènedioxy 4,5-séco 13 β-éthyl 17 β-acétoxy 17 α-éthynyl gonane ainsi formé que l'on isole ou non.

Le procédé de l'invention permet donc notamment de passer directement d'une énamine comme par exemple le 3-pyrrolidyl 13 β-éthyl 17 β-acétoxy 17 α-éthynyl gona 3,5-diène à l'oxime en 3 correspondante. S'il est classique de préparer les oximes en 3 à partir des cétones correspondantes et les cétones en 3 à partir d'énamines, le passage direct d'une énamine conjuguée à l'oxime correspondante sans formation intermédiaire du dérivé 3-céto Δ 4, n'avait jamais été réalisé jusqu'à présent.

L'analyse chromatographique permet d'affirmer qu'à aucun moment il n'y a formation du composé 3-céto Δ-4.

La réaction peut s'expliquer ainsi : il y a d'abord formation du sel d'immonium par action d'un acide sur l'énamine, puis action de l'hydroxylamine sur le sel d'immonium pour donner l'oxime selon le mécanisme suivant :

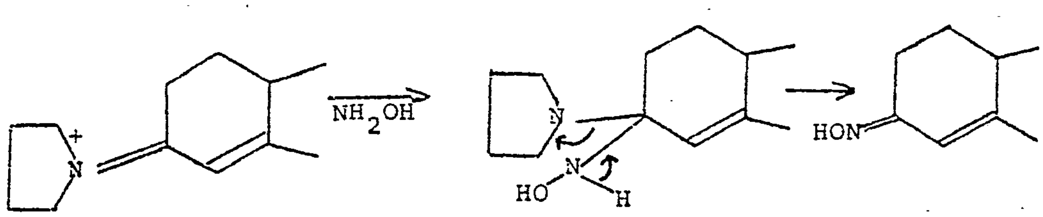


Le procédé de l'invention permet d'obtenir d'excellents rendements. C'est ainsi par exemple que l'on peut préparer la 3-oxime du 13 β-éthyl 17 β-acétoxy 17 α-éthynyl gon 4-en 3-one à partir du 3,5-dioxo 4,5-séco 13 β-éthyl 17 β-acétoxy 17 α-éthynyl gonane avec un rendement supérieur à 83 %.

Le procédé de l'invention est particulièrement intéressant sur le plan industriel car il livre un produit final pratiquement exempt de toute impureté.

Les exemples suivants illustrent l'invention sans toutefois la limiter.

Préparation du 3,5-dioxo 4,5-séco 13 β-éthyl 17 β-acétoxy 17 α-éthynyl gonane :

Stade A : 3,5-bis éthylènedioxy 4,5-séco 13 β-éthyl 17 β-acétoxy 17 α-éthynyl gonane.

On introduit 58 g de 3,5-bis éthylènedioxy 4,5-séco 13 β-éthyl 17 β-hydroxy 17 α-éthynyl gonane (préparé selon le procédé décrit dans le brevet israélien 28020) dans 174 $cm^3$ de toluène. Sous agitation et barbotage d'azote, on porte la suspension au reflux et distille 58 $cm^3$ de toluène. On ajoute à la solution obtenue 116 $cm^3$ d'anhydrique acétique, et porte à nouveau au reflux. Après les traitements usuels on obtient le produit recherché fondant à 165-166 °C.

Stade B : 3,5-dioxo 4,5-séco 13 β-éthyl 17 β-acétoxy 17 α-éthynyl gonane.

On ajoute 116 $cm^3$ d'eau, à la solution acétique obtenue au stade précédent, porte au reflux. On maintient le mélange réactionnel au reflux pendant trois heures et distille le toluène. On isole le précipité formé, l'essore, le lave et le sèche. On obtient ainsi 51,35 g du produit fondant à 123 °C.

Exemple 1 = 3-oxime de 13 β-éthyl 17 β-acétoxy 17 α-éthynyl gon 4-ène

Stade A : 3-pyrrolidyl 13 β-éthyl 17 β-acétoxy 17 α-éthynyl gon 3,5-diène.

On introduit 30 g du produit dont la préparation est donnée ci-dessus dans 150 $cm^3$ de méthanol. On ajoute ensuite 15 $cm^3$ de pyrrolidine. On maintient pendant 1 heure à 25-30 °C sous agitation et atmosphère d'azote. On essore, lave et sèche le précipité formé. On obtient ainsi 35 g de produit recherché fondant à 153 °C.
$\alpha^{20}_D = -276° \pm 3°$ (c = 0,5% diméthyl formamide)

Stade B : 3-oxime de 13 β-éthyl 17 β-acétoxy 17 α-éthynyl gon 4-ène

I) Préparation d'une suspension d'hydroxylamine :

On dissout 1,534 g de chlorhydrate d'hydroxylamine dans 60 $cm^3$ de méthanol. On ajoute à la solution ainsi obtenue 1,931 g d'acétate de sodium anhydre. On obtient ainsi une suspension d'hydroxylamine que l'on utilise telle quelle.

II) 3-oxime de 13 β-éthyl 17 β-acétoxy 17 α-éthynyl gon 4-ène.

On introduit sous agitation, et sous barbotage d'azote, 6 g du produit préparé au stade A dans 12 $cm^3$ d'acide acétique. On maintient la solution obtenue pendant 30 minutes sous agitation et la verse dans la suspension d'hydroxylamine préparée ci-dessus. On porte ensuite le mélange réactionnel au reflux sous agitation et barbotage d'azote pendant 3 heures. On ajoute ensuite à 20 °C, 180 $cm^3$ d'eau. On glace, essore, lave et empâte à l'eau. On obtient ainsi 5,327 g de produit que l'on purifie par recristallisation dans le méthanol. On obtient ainsi 4,795 g du produit recherché fondant à 223 °C. $\alpha^{20}_D = 40°$ à $+46°$(c = 0,5% chloroforme).

Exemple 2 : 3-oxime de 17 α-éthynyl 17 β-hydroxy estr 4-ène 3-one

Stade A : 3-pyrrolidyl 17-éthynyl 17 β-hydroxy estra 3,5(6)-diène

En opérant comme à l'exemple 1, stade A à partir du 3,5-dioxo 4,5-séco 17 α-éthynyl 17 β-hydroxy estrane, on obtient le composé recherché fondant à 186 °C.

Stade B : 3-oxime de 17 α-éthynyl 17 β-hydroxy estr 4-ène 3-one.

En opérant comme à l'exemple 1, stade B à partir du produit préparé au stade A du présent exemple, on obtient le produit recherché fondant à 112-114 °C.

**Revendications**

1. Procédé de préparation des composés de formule I :

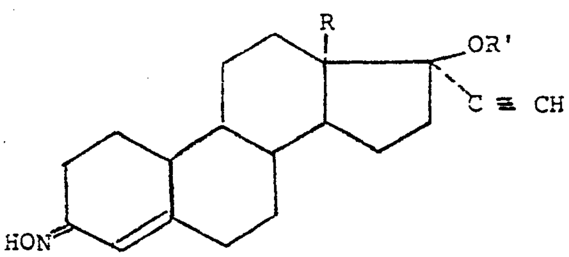

(I)

dans laquelle R représente un radical méthyle ou éthyle et R' un atome d'hydrogène ou un radical acyle renfermant de 2 à 8 atomes de carbone, caractérisé en ce que l'on soumet un composé de formule II :

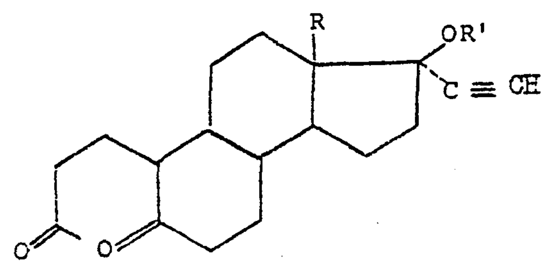

(II)

dans laquelle R et R′ conservent leur signification précédente à l'action d'une amine secondaire, puis à l'action d'un acide et enfin à celle de l'hydroxylamine ou d'un sel d'hydroxylamine, pour obtenir le composé recherché correspondant sous forme d'un mélange d'isomères syn et anti, que l'on sépare, si désiré, en chacun des isomères.

2. Procédé de préparation selon la revendication 1, caractérisé en ce que R représente un radical éthyle.

3. Procédé de préparation selon la revendication 1 ou 2, caractérisé en ce que R′ représente un radical acyle et notamment un radical acétyle.

4. Procédé de préparation selon la revendication 1, 2 ou 3, caractérisé en ce que l'on opère en milieu anhydre.

5. Procédé de préparation selon l'une quelconque des revendications 1 à 4, caractérisé en ce que l'on soumet le composé de formule II à l'action d'une amine secondaire, isole le composé ainsi obtenu que l'on soumet à l'action d'un acide, puis à celle de l'hydroxylamine ou d'un sel d'hydroxylamine pour obtenir le composé recherché sous forme d'un mélange d'isomères syn et anti que l'on sépare, si désiré, en chacun des 2 isomères.

6. Procédé de préparation selon l'une quelconque des revendications 1 à 5, caractérisé en ce que l'amine secondaire utilisée est choisie dans le groupe constitué par la pyrrolidine et la morpholine.

## Claims

1. Process for preparing the compounds of formula I:

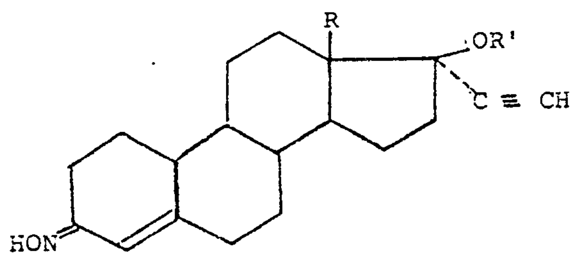

(I)

in which R represents a methyl or ethyl radical and R′ represents a hydrogen atom or an acyl radical containing from 2 to 8 carbon atoms, characterized in that a compound of formula II:

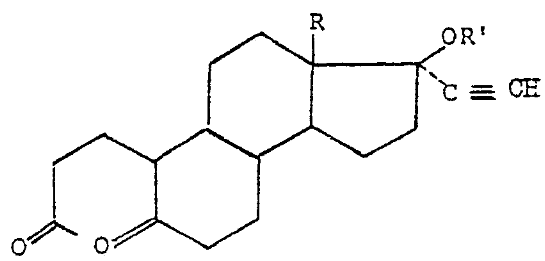

(II)

in which R and R′ keep their previous meaning, is subjected to the action of a secondary amine, then to the action of an acid and finally to that of hydroxylamine or of a hydroxylamine salt, to obtain the corresponding compound sought in the form of a mixture of syn and anti isomers which is separated, if desired, into each of the isomers.

2. Preparation process according to Claim 1, characterized in that R represents an ethyl radical.

3. Preparation process according to Claim 1 or 2, characterized in that R′ represents an acyl radical and especially an acetyl radical.

4. Preparation process according to Claim 1, 2 or 3, characterized in that work is carried out in anhydrous medium.

5. Preparation process according to any one of Claims 1 to 4, charactērized in that the compound of formula II is subjected to the action of a secondary amine, the compounds thus obtained is isolated and subjected to the action of an acid, then to that of hydroxylamine or of a hydroxylamine salt to obtain the compound sought in the form of a mixture of syn and anti isomers which is separated, if desired, into each of the two isomers.

6. Preparation process according to any one of Claims 1 to 5, characterized in that the secondary amine used is selected from the group constituted by pyrrolidine and morpholine.

**Ansprüche**

1. Verfahren zur Herstellung von Verbindungen der Formel I

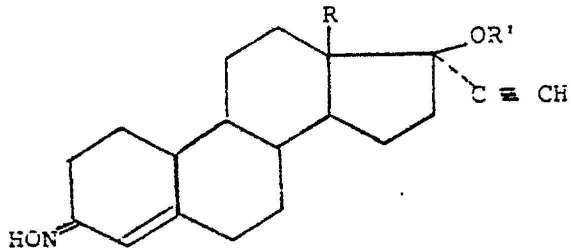

(I)

worin R einen Methyl- oder Äthylrest bedeutet und R′ ein Wasserstoffatom oder einen Acylrest mit 2 bis 8 Kohlenstoffatomen darstellt, dadurch gekennzeichnet, daß man eine Verbindung der Formel II

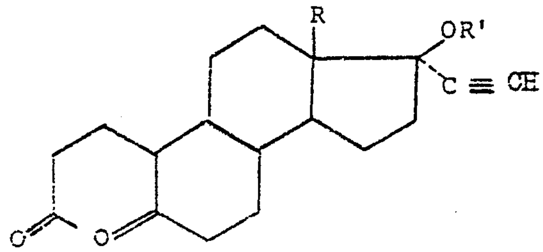

(II)

worin R und R′ die vorstehend angegebene Bedeutung besitzen, der Einwirkung eines sekundären Amins, danach der Einwirkung einer Säure und schließlich derjenigen von Hydroxylamin oder eines Hydroxylaminsalzes unterzieht, um die entsprechende gewünschte Verbindung in Form eines Gemisches der Syn- und Anti-Isomeren zu erhalten, das man gewünschtenfalls in ein jedes der Isomeren auftrennt.

2. Verfahren gemäß Anspruch 1, dadurch gekennzeichnt, daß R einen Äthylrest bedeutet.

3. Verfahren gemäß Anspruch 1 oder 2, dadurch gekennzeichnet, daß R′ einen Acylrest und insbesondere einen Acetylrest bedeutet.

4. Verfahren gemäß Anspruch 1, 2 oder 3, dadurch gekennzeichnet, daß man in wasserfreiem Milieu arbeitet.

5. Verfahren gemäß einem der Ansprüche 1 bis 4, dadurch gekennzeichnet, daß man die Verbindung der Formel II der Einwirkung eines sekundären Amins unterzieht, die so erhaltene Verbindung isoliert, diese der Einwirkung einer Säure, anschließend derjenigen von Hydroxylamin oder eines Hydroxylaminsalzes unterzieht, um die gewünschte Verbindung in Form eines Gemisches der Syn- und Anti-Isomeren zu erhalten, das man gewünschtenfalls in ein jedes der zwei Isomeren auftrennt.

6. Verfahren gemäß einem der Ansprüche 1 bis 5, dadurch gekennzeichnet, daß das verwendete sekundäre Amin ausgewählt wird unter Pyrrolidin und Morpholin.